# EUROPEAN PATENT APPLICATION

(11) **EP 4 800 093 A1**
(43) Date of publication of application: **02.09.2026**
(21) Application number: 24882327.0
(22) Date of filing: 21.10.2024
(51) Int. Cl.: C12M 3/00

(54) **CELL CULTURE SYSTEM, CULTURE TANK, AND CULTURE TANK CONNECTOR**

(30) Priority: 27.10.2023 JP 2023185007
(71) Applicant: Integriculture Inc., Tokyo 113-0033 (JP); Hamano Products Co., Ltd., Tokyo 131-0041 (JP)
(72) Inventor: KOWAKA, Masanobu, Tokyo 113-0033 (JP); NAGASHIMA, Hiroaki, Tokyo 131-0041 (JP)
(74) Representative: V.O.
(86) International application number: PCT/JP2024/037329
(87) International publication number: WO 2025/089214

(57) **Abstract**

The present invention provides a cell culture system which enables a simplification of the overall configuration thereof and which makes it possible to reduce the processing load on a control device. In a cell culture system S1 including: a plurality of culture tanks (100, 200 and 300) which are connected in series or in parallel via tubular members (60 to 63) and which store a culture medium (50); a pump (68), the number of which is less than the number of culture tanks, and which causes the culture medium to flow between the plurality of culture tanks; and a control device (70) which controls the pump, the plurality of culture tanks each has a pair of connection ports (3a, 3c) on side surfaces at a lower part thereof, and the tubular members are connected to the pairs of connection ports via connectors (10).

## Description

### Technical Field

The present invention relates to a cell culture system, a culture tank, and a connector for a culture tank.

### Background Art

In recent years, to achieve "Sustainable Development Goals (SDGs)," sustainable food production systems are actually being developed in countries around the world. In particular, "cell-based food" is being developed. In addition, for example, as a background art of the present technical field, Patent Literature 1 describes a cell culture system in which a plurality of culture tanks are connected to each other and a culture medium is circulated between the plurality of culture tanks.

### Citation List

### Patent Literature

Patent Literature 1: International Publication No. WO 2017/191691

### Summary of Invention

### Technical Problem

In a general configuration, when a plurality of culture tanks are connected using tubular members such as hoses, a culture medium is supplied and discharged from the upper portions of the culture tanks. However, when this configuration is applied to the cell culture system of Patent Literature 1, the same number of pumps as that of the culture tanks is required. The overall configuration of the cell culture system increases in size, and the plurality of pumps need to be simultaneously controlled, causing high processing load on the control device.

The present invention has been made in view of such circumstances described above, and the main object thereof is to provide a cell culture system that can simplify the overall configuration and reduce processing load on a control device. A further object of the present invention is to provide a culture tank and a connector for a culture tank that are preferable for the cell culture system.

### Solution to Problem

To achieve the above objectives, a first aspect of the present invention is a cell culture system. The system includes: a plurality of culture tanks connected in series or in parallel via tubular members to store a culture medium; pumps configured to have a smaller number than the number of the culture tanks to circulate the culture medium between the plurality of culture tanks; and a control device that controls the pumps. Each of the plurality of culture tanks has a pair of respective connection ports on lower portions of side surfaces thereof. The tubular members are respectively connected to the pair of connection ports via connectors.

To achieve the above objectives, a second aspect of the present invention is a culture tank that stores a culture medium. The culture tank includes a pair of connection ports respectively provided to lower portions of side surfaces thereof and a connector provided to each of the pair of connection ports to connect the tubular member thereto. The connector includes: a connector body that is inserted into the connection port and that forms a flow path therein through which the culture medium flows; a pair of packing members fitted over the connector body; and a nut fitted over the connector body to hold the pair of packing members. The connector body includes a flange portion provided to one end of the connector body and formed to have a larger outer diameter than that of the connection port, a fitting portion provided to the other end of the connector body to fit to the tubular member, and a threaded portion provided between the flange portion and fitting portion to be threadedly engaged with the nut. The connector body is inserted into the connection port to cause the flange portion to be positioned inside the culture tank and to cause the fitting portion to be positioned outside the culture tank. One of the pair of packing members is positioned between the inner wall of the culture tank and the flange portion, and the other is positioned between the outer wall of the culture tank and the nut.

To achieve the above objectives, a third aspect of the present invention is a connector for a culture tank, the connector being attached to a connection port of a culture tank that stores a culture medium to connect a tubular member. The connector includes a connector body being inserted into the connection port and forming a flow path therein through which the culture medium flows, a pair of packing members fitted over the connector body, and a nut fitted over the connector body to hold the pair of packing members. The connector body includes a flange portion provided to one end of the connector body and formed to have a larger outer diameter than the connection port, a fitting portion provided to the other end of the connector body to fit to the tubular member, and a threaded portion provided between the flange portion and fitting portion to be threadedly engaged with the nut. The connector body is inserted into the connection port to cause the flange portion to be positioned inside the culture tank, and to cause the fitting portion to be positioned outside the culture tank. One of the pair of packing members is positioned between the inner wall of the culture tank and the flange portion. The other is positioned between the outer wall of the culture tank and the nut.

### Advantageous Effects of Invention

According to the present invention, it is possible to provide a cell culture system that can simplify the overall configuration and reduce processing load on a control device. Further, according to the present invention, it is possible to provide a culture tank and a connector for a culture tank that are preferable for the cell culture system. Incidentally, problems, configurations, and effects other than those described above will be clarified by the following description of embodiments.

### Brief Description of Drawings

FIG. 1 illustrates a schematic overall configuration of a cell culture system according to First Embodiment.
FIG. 2 illustrates a configuration of the cell culture system of FIG. 1 viewed from the top surface.
FIG. 3 illustrates a schematic perspective view of a culture tank.
FIG. 4 illustrates a development view of a connector.
FIG. 5 illustrates a partial enlarged cross section of a state in which the connector is inserted into a connection port of the culture tank.
FIG. 6 illustrates a configuration of the cell culture system according to Second Embodiment viewed from the top surface. Description of Embodiments

Hereinafter, Embodiments of the present invention are explained using an example of culture of animal cells in reference to the drawings.

### (First Embodiment)

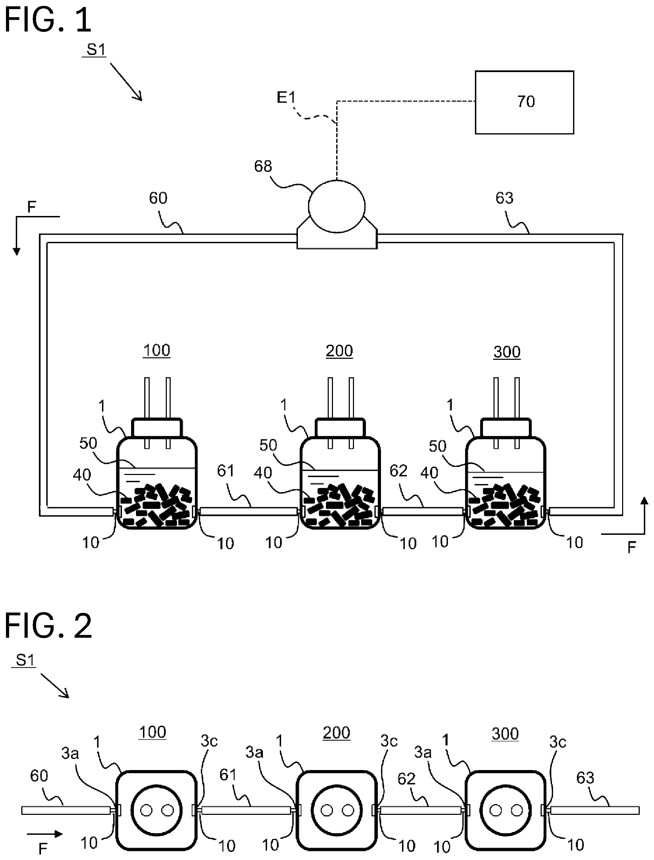

A cell culture system S1 according to First Embodiment of the present invention is explained. FIG. 1 illustrates a schematic overall configuration of the cell culture system S1 according to First Embodiment, and FIG. 2 illustrates a configuration of the cell culture system S1 of FIG. 1 viewed from the top surface. Incidentally, in FIG. 2, a pump 68 and a controller 70 are not illustrated.

As illustrated in FIGS. 1 and FIG. 2, the cell culture system S1 according to First embodiment arranges, for example, three culture tanks 100, 200, and 300 in series along the flow of a culture medium, and is formed in a loop by sequentially connecting the culture tanks 100, 200, and 300 via hoses 60 to 63 of examples of tubular members. Incidentally, the number of the culture tanks is not limited to three. In addition, instead of the hoses 60 to 63, pipes, tubes, and the like, may be used.

The culture tanks 100, 200, and 300 are, for example, containers made of stainless steel or glass, and store a culture medium 50 therein.

In the present embodiment, different animal cells are respectively supplied to the culture tanks 100, 200, and 300. The same animal cells may be supplied to some of or all the culture tanks 100, 200, and 300. Then the animal cells supplied to the culture tanks 100, 200, and 300 are attached to a scaffold 40 and cultivated to generate cell-based food. In addition, during the culture of the cell, the cells secrete useful components in the culture medium. These components are usable as a culture supernatant.

The scaffold 40 is for attaching animal cells thereto, has a sheet shape or a three-dimensional shape, and is formed of a porous or gel structure. The material for the scaffold 40, which is not particularly limited, may include a resin material such as PET and PP, a plant-based material, and an animal-origin material. In the present embodiment, the scaffold 40 is a plant-based or animal-origin edible scaffold. The scaffold 40 includes, for example, proteins and polysaccharides. The edible herein means a composition that may be ingested into a body safely. By use of this scaffold 40 as a substrate, animal cells are attached and proliferated to produce cell-based food.

The animal cells attached to the scaffold 40, that is, the animal cells supplied to the culture tanks 100, 200, and 300 include muscle-derived cells, skin-derived cells, intestine-derived cells, cardiac-derived cells, brain-derived cells, stomach-derived cells, embryonic membrane-derived cells, liver-derived cells, kidney-derived cells, and lung-derived cells. In particular, different types of the cells are introduced into the culture tanks 100, 200, and 300 to enable production of a more valuable culture supernatant.

Further, the cell culture system S1 includes one pump 68. The pump 68 is placed between the hose 60 and hose 63, and driven by an unillustrated motor. When the pump 68 is driven, the culture medium 50 is circulated from the culture tank 100 to culture tank 300 in the direction of the arrow F in the figure. Then, when the rotation rate of the motor changes, the flow rate of the pump 68 changes. That is, the circulation flow rate of the culture medium 50 flowing through the cell culture system S1 changes. Incidentally, the number of the pumps 68 is not limited to one. It is sufficient if the number is less than the number of the culture tanks.

The controller 70 is connected to the pump 68 (more specifically, to a motor that drives the pump 68) via electrical wiring E1. Further, the controller 70 is connected to unillustrated various sensors (a temperature sensor, a flow sensor, a pH detection sensor, etc.) via the electrical wiring, and can monitor the states of the culture tanks 100, 200, and 300 based on the sensor data inputted from the various sensors. Incidentally, the controller 70 is configured separately from the pump 68 in FIG. 1, and the controller 70 and pump 68 may be integrally configured.

The controller 70 is configured to include a CPU, a ROM and RAM as storage, and a calculation processing unit having other peripheral circuits. The ROM stores a control program. The CPU reads the control program to execute various processes. The controller 70 controls, e.g., the flow rate of the pump 68 based on inputted various pieces of information.

Next, the configuration for the culture tanks 100, 200, and 300 is explained in detail. Incidentally, since the culture tank 100 has the same configuration as the culture tanks 200 and 300, the culture tank 100 is explained hereinafter, and the culture tanks 200 and 300 are not explained.

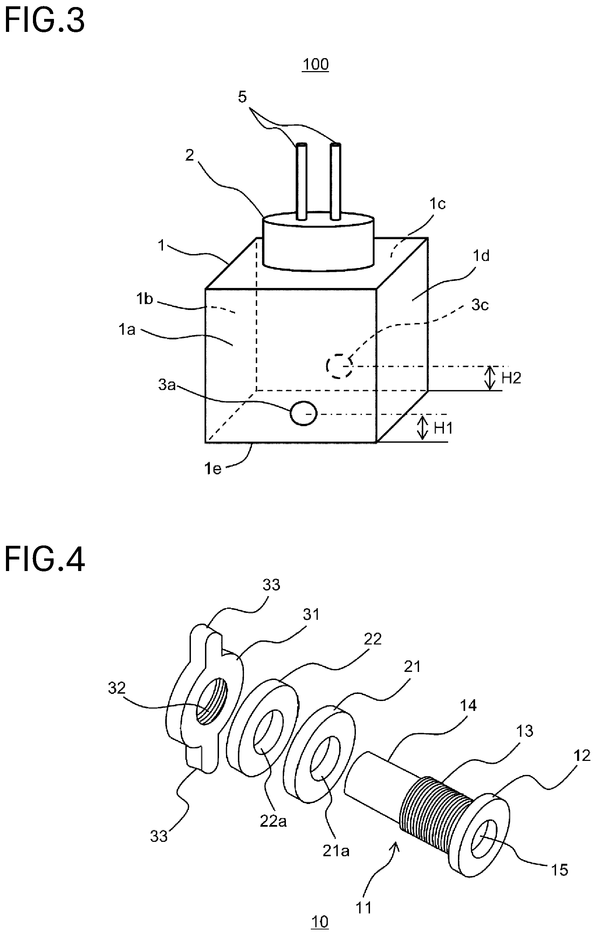

FIG. 3 illustrates a schematic perspective view of the culture tank 100. As illustrated in FIG. 3, the culture tank 100 includes a container body 1 and a lid 2 that covers an opening provided in the upper portion of the container body 1. The container body 1 is formed as a hollow cuboid. A pair of connection ports 3a, 3c are provided on opposing side surfaces 1a and 1c among side surfaces 1a to 1d. That is, the connection ports 3a, 3c are placed opposite each other. Incidentally, the connection port 3a is an inlet for the culture medium 50, and the connection port 3c is an outlet for the culture medium 50 (See FIG. 2).

The connection port 3a is provided at a height of the position H1 from the bottom surface 1e of the container body 1. Similarly, the connection port 3c is provided at a height of the position H2 from the bottom surface 1e of the container body 1. In the present embodiment, the position H1 and position H2 are the same in height. The position H1 and position H2 herein are preferably at, or lower than one-third of, the height of the container body 1 from the bottom surface 1e of the container body 1. Thus, the connection ports 3a, 3c are provided to the lower portions of the side surfaces of the container body 1 to ensure smooth circulation of the culture medium 50 even with one pump 68.

The lid 2 covers the opening of the container body 1 to seal the inside of the container body 1. Hose components 5 pass through the lid 2 to supply nutrients such as oxygen via the hose components 5.

Connectors 10 are inserted into the connection ports 3a, 3c, to which the hoses 60 to 63 are attached via the connectors 10 (See FIG. 2). The connector 10 is explained in detail in reference to FIG. 4. FIG. 4 illustrates a development view of the connector 10. As illustrated in FIG. 4, the connector 10 is configured to include a connector body 11, a pair of packing members 21, 22, and a nut 31.

The connector body 11 includes a flange portion 12 provided to one end of the connector body 11, a fitting portion 14 provided to the other end of the connector body 11, and a male threaded portion (threaded portion) 13 provided between the flange portion 12 and fitting portion 14. The connector body 11 includes therein a flow path 15 extending in the axial direction. The culture medium 50 flows through this flow path 15. The flange portion 12 is formed to have a larger outer diameter than each connection port 3a, 3c. The fitting portion 14 is inserted into, and fits to, each hose 60 to 63. Thus, the outer diameter thereof is formed to be generally the same as the inner diameter of each hose 60 to 63. The male threaded portion 13 is threadedly engaged with a female threaded portion 32 of the after-described nut 31.

The pair of packing members 21, 22 are made of, e.g., a silicon resin. The pair of packing members 21, 22 are fitted over the connector body 11. Thus, each inner diameter of holes 21a, 22a of the packing members 21, 22 is slightly larger than the outer diameter of the male threaded portion 13 of the connector body 11.

The nut 31 is fitted over the connector body 11. The nut 31 has a pair of projections 33 on the periphery thereof. The projections 33 are for hooking fingers of an operator when the nut 31 is rotated. The female threaded portion 32 of the nut 31 is threadedly engaged with the male threaded portion 13 of the connector body 11 to hold the pair of packing members 21, 22 between the nut 31 and flange portion 12.

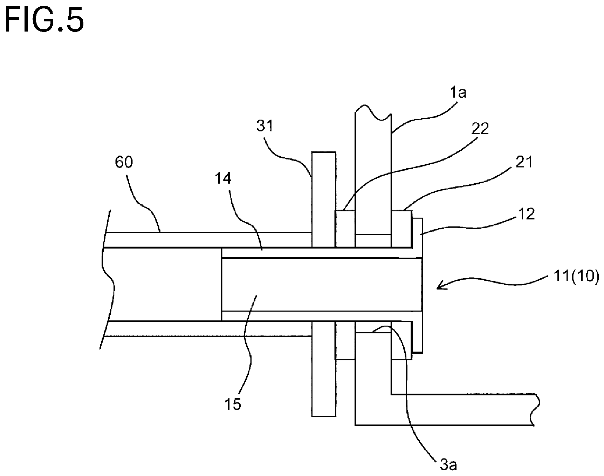

Next, an attachment method for the connector 10 is explained. FIG. 5 illustrates a partial enlarged cross section of a state in which the connector 10 is inserted into the connection port 3a of the culture tank 100. As illustrated in FIG. 5, in the state where the packing member 21 is fitted over the connector body 11, when the connector body 11 is inserted into the connection port 3a from inside (from inside the container body 1) outwardly, the flange portion 12 abuts against the side surface 1a of the container body 1 via the packing member 21. That is, the packing member 21 is sandwiched between the flange portion 12 and the side surface 1a of the container body 1.

Next, after the packing member 22 is fitted over the fitting portion 14 of the connector body 11, the fitting portion 14 being exposed outside the container body 1 from the connection port 3a, the nut 31 is fitted over the connector body 11 and tightened. Then, the pair of packing members 21, 22 seal the connection port 3a.

In this state, the hose 60 is inserted onto the fitting portion 14 of the connector body 11 and secured. Thus, the hose 60 communicates with the container body 1 via the flow path 15 of the connector 10. That is, the culture medium 50 can flow from the hose 60 into the container body 1. Incidentally, the connector 10 is attached to the connection port 3c in the same way as above.

According to the present embodiment configured as above, the following effects are achievable.

For example, since the hoses 60 to 63 are connected to the connection ports 3a, 3c of the lower portions of the culture tanks 100, 200, and 300, the culture medium 50 does not need to be pumped up to a higher position than the culture tanks when the culture medium 50 is circulated. Thus, the culture medium 50 can be circulated using a smaller number of pumps than the number of culture tanks (three in the present embodiment), especially one pump 68. Therefore, the overall configuration of the cell culture system S1 can be simplified.

In addition, since the controller 70 has only to control one pump 68, the control processing load is further reduced in comparison with the case where a plurality of pumps are controlled. Thus, the present embodiment contributes to achievement of sustainable development goals (SDGs). In addition, the configuration for the culture tanks 100, 200, and 300 themselves is simple, and incorporated in various devices to enable easy construction of a cell culture system, thereby providing high scalability.

In addition, since the connection ports 3a, 3c are placed opposite each other, the culture tanks 100, 200, and 300 are easily arranged in a line, and routing of the hoses 60 to 63 is easy. Further, since the heights H1, H2 of the connection ports 3a, 3c are the same, the routing of the hoses 60 to 63 is possible without considering the directions of the culture tanks 100, 200, and 300. Thus, efficiency in assembly of the cell culture system S1 is improvable.

In addition, since the connectors 10 are inserted into the connection ports 3a, 3c, and the hoses 60 to 63 can be connected to the container bodies 1 via the connectors 10, the culture tanks 100, 200, and 300 are easily connected to each other. In particular, when the flange portion 12 of the connector 10 abuts against the inner wall of the container body 1 via the packing member 21, and then the nut 31 is tightened to cause the packing member 22 to abut against the outer wall of the container body 1, the attachment of the connector 10 to each connection port 3a, 3c is completed. At this point, since the packing members 21, 22 seal the connection ports 3a, 3c, there is no concern about leakage of the culture medium 50. Then, when each hose 60 to 63 fits to the fitting portion 14, the routing of the hoses 60 to 63 is completed. In such a way, by use of the connector 10, the cell culture system S1 can be easily constructed. In addition, when a pipe and a tube are used instead of the hoses 60 to 63, it is only necessary to change the shape of the fitting portion 14 of the connector body 11, improving the versatility.

### (Second Embodiment)

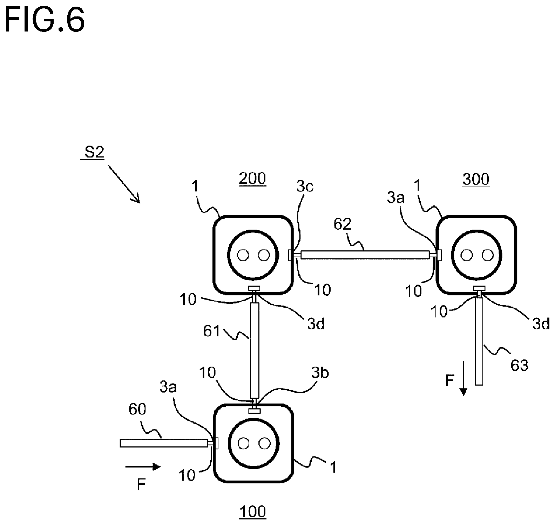

Next, a cell culture system S2 according to Second Embodiment of the present invention is explained. FIG. 6 illustrates a configuration of the cell culture system S2 according to Second Embodiment viewed from the top surface. Incidentally, in FIG. 6, the pump 68 and the controller 70 are not illustrated.

As illustrated in FIG. 6, in Second Embodiment, the positions of the connection ports provided to the culture tanks 100, 200, and 300 are different from those in First Embodiment. Specifically, for the culture tank 100, the connection port 3a and the connection port 3b are respectively provided to the side surface 1a and the side surface 1b (See FIG. 3) of the container body 1. That is, the connection port 3a and the connection port 3b are not provided at positions facing each other, and the connection port 3b is provided at a position rotated 90 degrees from the connection port 3a in top view. The culture tanks 200 and 300 are configured in the same way as above. Incidentally, the connection ports 3a, 3b, 3c, and a connection port 3d all are positioned at the same height.

According to Second Embodiment configured in such a way, the same advantageous effect as First Embodiment is obtainable. Further, since the culture tanks 100, 200, and 300 are placeable without being in line, the layout flexibility is advantageously improved.

Incidentally, the present invention is not limited to the above embodiments, and may be variously modified without departing from the outline of the present invention. All the technical matters contained in the technical concept described in Claims are objects of the present invention. The above embodiments describe preferred examples. Those skilled in the art can achieve various alternative examples, amended examples, modified examples, or improved examples from the disclosed contents of the present specification. These are included in the technical range described in the appended Claims.

For example, the present invention is applicable as a device that cultivates cells other than animal-origin cells (for example, plant-based cells).

In addition, instead of configuring the cell culture system by connecting the culture tanks 100, 200, and 300 in series, these culture tanks may be connected in parallel to configure the cell culture system.

The height positions of the connection ports 3a, 3c can be configured to be different from each other. In this case, the connection port 3a on the inlet side for the culture medium 50 is preferably higher than the connection port 3c on the outlet side (that is, H1 > H2). In such a way, the convection of the culture medium 50 is made large due to the head difference, and the culture medium 50 is distributed evenly inside the culture tank 100. Consequently, more nutrients and oxygen can be supplied to cells inside the culture tank 100.

Furthermore, the positions of the connection ports 3a, 3c are changeable in the width direction. In this case, in FIG. 3, for example, the connection port 3a on the inlet side for the culture medium 50 is provided leftward of the culture tank 100, and the connection port 3c on the outlet side is provided rightward of the culture tank 100. The distance between the inlet and outlet is thus made long. Consequently, the convection of the culture medium 50 is made large, the culture medium 50 spreads more evenly inside the culture tank 100, and more nutrients and oxygen can be supplied to cells inside the culture tank 100.

In addition, the connector 10 may be provided to the upper portion of the container body 1 or on the lid 2 to be usable for gas exchange for the container body 1.

The container body 1 herein is not limited to a hollow cuboid shape. For example, a cylindrical container body or a container body having another shape may be applied to the present invention. In this case, a pair of connection ports, which are to be placed opposite each other, may be placed generally opposite each other based on the shape of the container.

In addition, a material for the pair of packing members 21, 22 is not limited to silicone. Any packing members made of a material capable of ensuring the sealed condition of the container body 1 may be used. In addition to the pair of the packing members 21, 22, metal washers may be used.

### Reference Signs List

1: container body
1a to 1d: side surface
2: lid
3a to 3d: connection port
5: hose component
10: connector
11: connector body
12: flange portion
13: male threaded portion (threaded portion)
14: fitting portion
15: flow path
21, 22: packing member
31: nut
32: female threaded portion
33: projection
50: culture medium
60 to 63: hose (tubular member)
68: pump
70: controller (control device)
100, 200, 300: culture tank
S1, S2: cell culture system

## Claims

1. A cell culture system comprising:
a plurality of culture tanks being connected in series or in parallel via a tubular member and storing a culture medium;
a pump being configured of a smaller number than the number of the culture tanks and circulating the culture medium between the plurality of culture tanks; and
a control device that controls the pump,
wherein each of the plurality of culture tanks has a pair of respective connection ports on lower portions of side surfaces thereof, and
the tubular member is connected to each of the pair of the connection ports via a connector.

2. The cell culture system according to claim 1 wherein
the pair of connection ports are arranged at positions facing each other.

3. The cell culture system according to claim 1 or 2 wherein
the pair of connection ports are positioned at the same height.

4. The cell culture system according to claim 1 wherein
the connector includes:
a connector body being inserted into the connection port and forming a flow path therein through which the culture medium flows;
a pair of packing members fitted over the connector body; and
a nut being fitted over the connector body and holding the pair of packing members,
the connector body includes:
a flange portion being provided to one end of the connector body and being formed to have a larger diameter than that of the connection port; a fitting portion being provided to the other end of the connector body and fitting to the tubular member; and a threaded portion being provided between the flange portion and the fitting portion and being threadedly engaged with the nut,
the connector body is inserted into the connection port to cause the flange portion to be positioned inside the culture tank and cause the fitting portion to be positioned outside the culture tank,
one of the pair of packing members is placed between an inner wall of the culture tank and the flange portion, and the other is placed between an outer wall of the culture tank and the nut.

5. A culture tank that stores a culture medium, comprising:
a pair of connection ports respectively provided to lower portions of side surfaces thereof; and
a connector being provided to each of the pair of connection ports and connecting a tubular member thereto,
the connector including:
a connector body being inserted into the connection port and forming a flow path therein through which the culture medium flows;
a pair of packing members fitted over the connector body; and
a nut being fitted over the connector body and holding the pair of packing members,
the connector body including:
a flange portion being provided to one end of the connector body and having a larger outer diameter than that of the connection port; a fitting portion being provided to the other end of the connector body and fitting to the tubular member; and a threaded portion being provided between the flange portion and the fitting portion and being threadedly engaged with the nut, wherein
the connector body is inserted into the connection port to cause the flange portion to be positioned inside the culture tank and cause the fitting portion to be positioned outside the culture tank,
one of the pair of packing members is placed between the inner wall of the culture tank and the flange portion, and the other is placed between the outer wall of the culture tank and the nut.

6. A connector for a culture tank, the connector being attached to a connection port of the culture tank and connecting a tubular member thereto, the culture tank storing a culture medium, the connector comprising:
a connector body being inserted into the connection port and forming a flow path therein through which the culture medium flows;
a pair of packing members fitted over the connector body; and
a nut being fitted over the connector body and holding the pair of packing members,
the connector body including:
a flange portion being provided to one end of the connector body and having a larger outer diameter than the connection port; a fitting portion being provided to the other end of the connector body and fitting to the tubular member; and a threaded portion being provided between the flange portion and the fitting portion and being threadedly engaged with the nut,
wherein the connector body is inserted into the connection port to cause the flange portion to be positioned inside the culture tank and to cause the fitting portion to be positioned outside the culture tank,
one of the pair of packing members is positioned between the inner wall of the culture tank and the flange portion, and the other is positioned between the outer wall of the culture tank and the nut.
